# EUROPEAN PATENT SPECIFICATION

(11) **EP 2 413 808 B1**
(45) Date of publication and mention of the grant of the patent: **28.10.2015**
(21) Application number: 10712106.3
(22) Date of filing: 31.03.2010
(51) Int. Cl.: A61B 10/02

(54) **SURGICAL INSTRUMENT**
OPERATIONSINSTRUMENT
INSTRUMENT CHIRURGICAL

(30) Priority: 31.03.2009 GB 0905594
(43) Date of publication of application: 08.02.2012
(73) Proprietor: Maidstone & Tunbridge Wells NHS Trust, Kent ME16 9QQ (GB)
(72) Inventor: SCOTT, Richard, Mark, Kent ME9 8HX (GB); HALL, Stephen, Addison, Kent TN3 9HX (GB)
(74) Representative: Henderson, Helen Lee
(86) International application number: PCT/GB2010/000633
(87) International publication number: WO 2010/112850

(56) References cited:
- WO-A1-2005/023122
- US-A1- 2005 113 854

## Description

This invention relates to a surgical instrument and, in particular, to a surgical instrument for removing a tissue sample from the body of a human or animal.

It is known to perform a biopsy on a human or an animal, in order to remove a sample of tissue or the like, for further analysis. In known methods of performing a biopsy, a surgeon or doctor would typically use forceps, or a similar device, enabling him/her to grip a piece of tissue from or near to the area from which a sample is to be taken. Using a different device or, in some cases, the same device, the tissue is cut, and a sample of the tissue from the desired area is removed.

Often, it is required to take a sample from an area of a body which is not easily accessible to a surgeon or doctor, for example the cervix. In such a case, a colposcopist is required take a sample from the cervix of a person or animal. A biopsy of this kind is typically performed without anaesthetic, and it is, therefore, important that the biopsy is performed as quickly and as painlessly as possible, in order to cause as little discomfort to the patient as possible.

A known device for performing biopsies is disclosed in US6083150, in which an opposing pair of cup-shaped jaws are pivotally connected to a shaft. In use, a user causes the cups to pivot towards one another, cutting into the tissue from which a sample is to be taken, until the cups engage one another, forming an enclosure, containing the sample. In this device, the cups 'bite' into the tissue as they close towards one another, sometimes causing the tissue to tear, rather than cutting the tissue cleanly. The tissue to be cut may also move away from the cups, rather than be cut, even if the instrument incorporates a spike. A sample obtained from a biopsy performed using a device such as this is often damaged and of a non-uniform shape, resulting in analysts being uncertain as to the orientation from which the sample was excised from the body. Furthermore, the 'biting' of the cups into the tissue can be painful and traumatic for a patient, causing discomfort.

Another biopsy device is disclosed in US5074311. In this device, an annular cutting blade is provided in a hollow housing, and may be advanced through an aperture in the end of the housing to make an annular cut in the tissue from which a sample is required to be taken. An inner tube is then advanced, to cause flexible tangs to project through the aperture, and into the tissue. The tangs are urged inwards, within the annular cut made by the annular blade, to sever the sample, so that it can be removed from the body. A disadvantage of this device is that it contains many moving parts, meaning the cost of producing it will be more than a device having fewer moving parts. In addition, more incisions are made in the tissue than are required to take the sample; the annular blade first cuts into the tissue; then the tangs make a second cut into the tissue to sever the sample from the body. A patient from whom a sample is being taken could find this very painful and uncomfortable. Furthermore, with so many moving parts, the device is difficult to clean and sterilise. Such a device is used repeatedly, to take samples from different patients. It is therefore imperative that the device is cleaned thoroughly, and sterilised to an acceptable medical standard. It is entirely possible that a piece of tissue excised from the patient could become inadvertently lodged between the annular blade and the tangs, and could remain in the device following sterilization. There is, therefore, an increased risk of cross contamination and, potentially, a risk of transferring diseases between patients.

In US 2005/0113854, a cervical conization device is disclosed. This device is used to remove a conical section of tissue from a cervix for pathological examination of a region, as opposed to a specific site. A circular knife comprising a number of curved blades is disposed within, and movable along a longitudinal axis of a hollow housing. A barbed stabilization rod extends from an opening in the housing. In use, the stabilization rod is first inserted into the tissue intended to be removed. The barbs hold the tissue in place. The knife is then caused to emerge from the opening in the housing, the blades of the knife are urged towards one another to form a conical enclosure containing the sample to be removed. Due to the insertion of the stabilization rod, the sample is damaged before it is even removed. If it is important to analyse the epithelium of the sample, then use of a device of this type may not be suitable. Furthermore, the blades of the knife are curved, so as to form a conical shape when urged together. Forming a blade having this shape is expensive, and it is often difficult to form the blade in the exact shape required. Any minor defect in the blade could result in the device not functioning correctly, or a sample being damaged during its removal from a body of tissue. In fact, it is unlikely that the blades of the invention disclosed in US2005/0113854 would even meet at all, due to the blades having curved edges. After they pass the annular shoulder, they would converge in straight lines. The blades would not, therefore, meet along their entire length. Thus, it is likely that there would be some tearing of the tissue being removed.

It is an aim of the present invention to provide an improved surgical instrument, for extracting tissue from a body of tissue, overcoming, or at least mitigating, the above-mentioned problems of the devices known in the art.

In a first aspect of the present invention, a surgical instrument for removing a sample from a body of tissue is provided.

In a first embodiment of the first aspect of the present invention, the surgical instrument comprises a support structure and a knife arranged to cut into the body of tissue, the knife having at least a first blade, a second blade and a third blade, each blade having two cutting edges defining an apex, and the knife being movable between a first configuration in which the blades are substantially aligned along a longitudinal axis of at least part of the support structure, and a second configuration in which the blades define the walls of a pyramidal enclosure for containing the sample.

In use, a surgeon or other practitioner, positions the instrument against the tissue to be sampled. The blades are advanced from the first configuration into the second configuration and cut through the tissue. In the first configuration, the blades are open, the cutting edges of each blade being positioned apart or away from the cutting edges of the neighbouring blades. In the second configuration, the blades are closed, each cutting edge of each blade meeting a cutting edge of the neighbouring blade. The two cutting edges of each blade meet at an apex. In the closed configuration, the apexes meet, forming the apex of a pyramid. The cutting edges meet, forming the edges of the pyramid and the blades forming the sides. As the blades are advanced towards the second configuration, they cut through the tissue. The sample is neat as the tissue is cut, rather than torn as in prior art devices. Additionally, the sample can be cut in one motion. Certain prior art devices require two cutting movements, boring a sample and then slicing or tearing it away from the remaining tissue. Furthermore, the instrument can be positioned directly over the tissue from which a sample is to be taken, rather than positioning the instrument slightly to one side, which is required by some existing instruments.

In the first configuration, each blade is flat, or substantially flat. When the blades are advanced into the second configuration, they are urged or biased inwards such that the position of the base behind the pair of cutting surfaces on each blade is precisely defined and the course of the apex of each blade is precisely defined. This is achieved by each flexible blade pressing firmly against its biasing member. Only the end of each blade having the cutting edges is advanced into the tissue, each in a single plane, and it is these parts of the blades which form the pyramidal enclosure to contain the sample. When each blade cuts into, and travels through, the tissue, it moves in a straight line, thereby cutting, not tearing the tissue. The cutting of the tissue is carried out only by the blades. The pyramidal shape that the blades form in the second configuration means that no additional cutting is required to detach the sample from the body of tissue from which it is taken. The single movement of the blades through the tissue, each in a straight line and plane, results in the complete separation and enclosure of a sample from the body of tissue, allowing its ready removal.

Since the apexes of the three, preferably four, or more opposing blades grip the tissue as the blades are advanced, the need for a separate member to grip the tissue is eliminated or at least reduced.

Since the portions of the blades that perform the cutting of the tissue are flat as they pass through the tissue, the sample that is obtained also has flat sides. Thus, the shape of a sample obtained using the device is pyramidal, or at least substantially pyramidal. The resulting pyramidal sample has flat sides and, therefore, the sample can be handled easily once removed. The flat sides of the sample enable it to be placed stably on a surface, without the risk of it rolling around, as may happen with a dome-shaped or cylindrical-shaped sample.

In the second configuration, as mentioned above, the apex of each blade meets the apex of each other blade. Thus, in the transition from the first configuration to the second configuration, the blades cut and sever the sample fully, resulting in a cleanly-cut pyramidal sample, with no tearing. This has the important additional advantage that the sample obtained is relatively undamaged and, as a result, is more useful diagnostically.

When a sample of tissue is taken, the sample is retained in the pyramidal enclosure made by the blades. The sample is itself pyramidal in shape. This is particularly useful, as it allows the orientation of the sample to be determined easily after removal. For example, in an embodiment in which the instrument has four blades, a sample is removed which has the shape of a four-sided pyramid. The epithelium forms the square base of the pyramid, and the user can determine, from the sample's orientation once it has been removed, how the sample was oriented before its removal. Following removal, the sample may be cut into sections which are then fixed on a slide for microscopic analysis. The flat sided sample obtained by the device is easier to cut into regular sections.

The instrument may further comprise a biasing member arranged to urge each blade towards the central axis of the support structure.

The instrument further may comprise actuation means, capable of causing the knife to move between the first configuration and the second configuration. The instrument preferably further comprises locking means for preventing movement of the knife between the first configuration and the second configuration and/or between the second configuration and the first configuration. The actuation means may be such that the movement of the blades between the first configuration and the second configuration is achieved in one motion. A locking means allows the blades to be held in a safe position until a user of the instrument is ready to take a sample and/or to hold the blades in the closed configuration once a sample has been taken. It is undesirable for the blades to advance to a cutting position before the instrument is in the correct position, to prevent premature damage being caused to tissue other than the tissue to be removed. Similarly, it is undesirable for the blades to release the sample before the instrument has been fully removed from the site from which the sample is being taken.

According to a second embodiment of the first aspect of the invention, the instrument comprises a plunger disposed in, and being axially movable in a longitudinal direction along, the support structure, the plunger being arranged to move the knife between the first configuration and the second configuration, and/or between the second configuration and the first configuration.

According to a third embodiment of the first aspect of the invention, each blade remains in the same plane in transition between the open and closed configurations. In this embodiment, in the open configuration, the blades are substantially outside the support structure. The blades are not required to bend and, therefore, can be formed of a stronger, more rigid material.

Preferably, each blade is diametrically opposite another blade. More preferably, the knife further comprises a fourth blade, the fourth blade being arranged such that the fourth blade is diametrically opposite to the second blade, and the third blade being diametrically opposite to the first blade. An advantage of having each blade arranged diametrically opposite to another blade, for example as can be achieved with any even number of blades, is that the blades grip the tissue evenly as they enter. The gripping action performed by the blades as they move towards one another ensures that, as the blades enter the tissue, the tissue is not urged away from the instrument and, therefore, the blades are able to make a cleaner cut in the tissue, with less tearing.

Advantageously, the support structure may include an angled region, such that the axis of the knife is offset from the axis of at least part of the support structure, or at an angle to the axis of the handle-end of the support structure. Alternatively, the support structure is angled close to the knife, or is capable of being angled or bent. An advantage of the instrument including a kinked, or angled, support structure, or of the support structure being capable of being bent, is that it can be used to obtain a sample from sites that are less directly accessible to a user of the instrument.

Preferably, the knife is pivotable relative to the axis of the support structure. Specifically, it is preferable that the knife is pivotable through an angle of between 0 and 90 degrees from the axis of the support structure. More preferably, the knife is pivotable through an angle of between 0 and 45 degrees from the axis of the support structure. Conveniently, the instrument may further comprise a securing means for securing the knife in a pivoted position relative to the support structure. It is important that, when obtaining a sample, the instrument is held approximately perpendicular to the surface of the tissue from which a sample is to be taken. Depending on the location of the sample to be taken, this is not always achievable. Therefore, it is advantageous to be able to pivot the knife relative to the tubular member, to enable a user to obtain a sample from an area that is not directly accessible.

Advantageously, the instrument is constructed from materials that are readily available and available at low cost. Ideally, the instrument is easy and inexpensive to manufacture, such that it is cost-effective to dispose of the instrument once it has been used to remove one or more samples from a single patient. Alternatively, if it is desirable to reuse the instrument, it is advantageously suitable for being cleaned or sterilized using known cleaning or sterilization methods.

Preferably, the construction of the instrument is such that a user has a clear line of sight through the instrument, allowing him or her to view the surface of the tissue from which a sample is to be taken, until and while the blades are moved to the second, closed configuration.

In a second aspect of the present invention, a knife assembly is provided. The knife assembly comprises at least a first blade, a second blade and a third blade, each blade having two cutting edges defining an apex, and the knife assembly being arranged for use with the surgical instrument described herein. The blades are preferably arranged to be moveable between a first and a second configuration, wherein in the second configuration, the blades are arranged to form a pyramid.

The invention will now be described in greater detail, by way of example, with reference to the drawings, in which:-
Figure 1 is a sectional view of a surgical instrument constructed in accordance with a first embodiment of the invention;
Figure 2 is an enlarged perspective view of a part of the surgical instrument of Figure 1;
Figure 3 is a perspective schematic view of a part of the surgical instrument of Figure 1, in a first configuration;
Figure 4 is a sectional view through line A-A of Figure 3;
Figure 5 is a perspective schematic view of a part of the surgical instrument of Figure 1, in a second configuration;
Figure 6 is a sectional view through line B-B of Figure 5;
Figure 7 is a schematic perspective view of a sample obtained using the surgical instrument of Figure 1;
Figure 8 is a schematic view of a part of a surgical instrument constructed in accordance with a second embodiment of the invention;
Figures 9, 10, 11 and 12 are schematic views of the surgical instrument of Figure 8 in various progressive states of use; and
Figures 13 and 14 are schematic views of a surgical instrument constructed in accordance with a third embodiment of the invention.

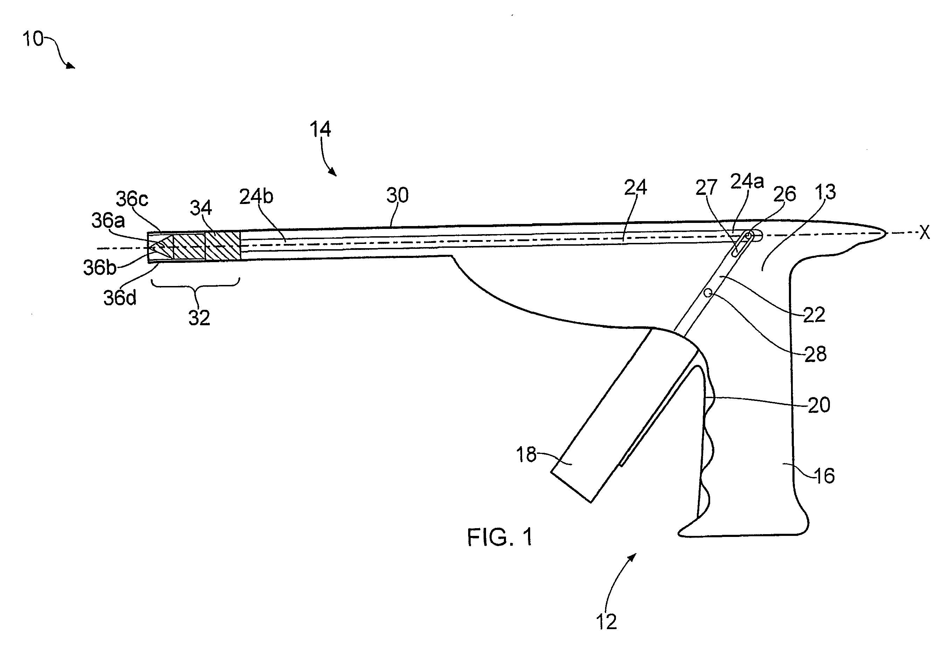

Referring to the drawings, a surgical instrument 10 constructed in accordance with a first embodiment of the invention is shown in Figure 1. The surgical instrument 10 comprises a handle 12, a body 13 and a shaft 14. The part of the instrument 10 constituting the handle 12 is a standard handle known to those skilled in the art of surgical instrument design. In this particular embodiment, the handle 12 comprises a grip 16, and a trigger 18 connected to the body 13, and pivotally movable relative to the grip. The trigger 18 is moveable between a first rest position and a second engaged position. A leaf spring 20 acts as a resilient member, urging the trigger 18 to its first position.

A trigger rod 22 extends linearly from the trigger 18. A driving rod 24 has a proximal end 24a and a distal end 24b, and is connected, at the proximal end, to the trigger rod 22 by a first pivot 26. The first pivot 26 passes through, and is slidable, along, a slot 27 formed in the trigger rod 22. A second pivot 28 is positioned part way along the trigger rod 22, arranged to allow pivotal movement of the trigger rod and trigger 18.

The shaft 14 of the instrument 10 comprises a support structure 30. The support structure 30 may be any suitable structure capable of supporting the components of the instrument 10, for example a wire frame or a hollow tube of any cross-sectional shape, such as, but not limited to, square, triangular or circular. In this embodiment, the support structure 30 is a hollow tube having a substantially square cross-section. The hollow tube 30 defines a longitudinal axis X. The driving rod 24 extends into, and is movable along the longitudinal axis X of, the hollow tube 30. A knife member 32 is disposed within the hollow tube 30, and is connected to the distal end 24b of the driving rod 24. The knife member 32 comprises a knife base 34 and four blades 36a, 36b, 36c, 36d extending from the knife base 34.

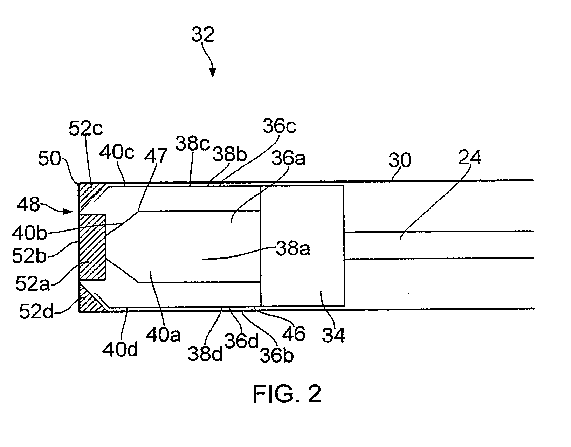

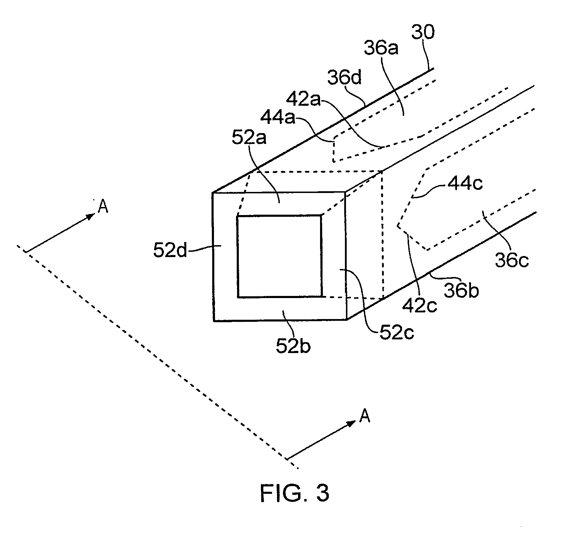

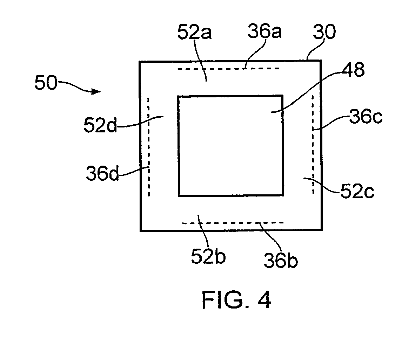

Figures 2 to 4 show the knife member 32 in more detail. In Figure 2, it can be seen that the blades 36a, 36b, 36c, 36d extend from the knife base 34, along the inside walls of the hollow tube 30. Within the hollow tube 30, the blades 36a, 36b, 36c, 36d remain substantially straight. A first blade 36a is diametrically opposite to, and substantially parallel to a second blade 36b, and a third blade 36c is diametrically opposite to, and substantially parallel to, a fourth blade 36d. Each of the blades 36a, 36b, 36c, 36d includes an elongate part 38a, 38b, 38c, 38d and a cutting part 40a, 40b, 40c, 40d. Each cutting part 40a, 40b, 40c, 40d is substantially triangular in shape, thereby forming two cutting edges 42a, 44a; 42b, 44b; 42c, 44c; 42d, 44d (see Figure 3) on each blade 36a, 36b, 36c, 36d. Each pair of cutting edges 42a, 44a; 42b, 44b; 42c, 44c; 42d, 44d defines an apex, which constitutes a sharp point. The arrangement of the blades 36a, 36b, 36c, 36d can be seen more clearly in Figures 3 and 4.

A person skilled in the art will appreciate that each of the blades 36a, 36b, 36c, 36d may be formed differently, for example without an elongate part 38.

In this particular embodiment, the blades 36a, 36b, 36c, 36d are formed from stainless steel. However, it will be apparent to a person skilled in the art that the blades 36a, 36b, 36c, 36d could be formed from any material suitable for making surgical incisions. The instrument 10 in this embodiment is made from any material suitable for the manufacture of surgical instruments, for example, plastics, metal, or other materials. The whole instrument 10 may be manufactured from the same material, or various components may be manufactured from different materials.

Referring again to Figure 2, a core member 46 extends from the knife base 34, along the interior of the hollow tube 30, and within the elongate part 38a, 38b, 38c, 38d of each blade 36a, 36b, 36c, 36d. The core member 46 is fixedly attached to the knife base 34, and is moveable along the longitudinal axis X of the hollow tube. The core member 46 is substantially cuboidal in shape, and is arranged in such a way that the blades 36a, 36b, 36c, 36d are held substantially straight within the tube 30. A leading surface 47 of the core member 46 is located at approximately the same position along the tube 30 as the point where the elongate parts 38a, 38b, 38c, 38d of the blades 36a, 36b, 36c, 36d become the cutting parts 40a, 40b, 40c, 40d.

Again, a person skilled in the art will appreciate that the knife member 32 may be formed in a different manner, for example without a core number 46, but with some other means for advancing the blades.

An opening 48 is formed at the end 50 of the tube 30. At the opening, a biasing member 52a, 52b, 52c, 52d is formed on the inner surface of each wall of the tube 30 (see also Figures 3 and 4). The biasing members 52a, 52b, 52c, 52d project inwards from each wall, and are tapered down into the tube 30, to form a slope.

In use, the surgical instrument 10 is held in a hand of a user; typically a doctor, surgeon, or colposcopist. Referring again to Figure 1, the user holds the grip 16 in his or her hand, in such a way that the trigger 18 is operable using the index finger, or the index finger and the middle finger. The instrument 10 is positioned such that the opening 48 at the end 50 (see Figure 2) of the tube 30 is close to, or gently touching; the surface of the tissue from which is sample is to be removed. Although not essential, it is beneficial to position the instrument such that the tube 30 is substantially perpendicular to the surface of the tissue. The user squeezes the trigger 18, such that the trigger and trigger rod 22 rotate about the second pivot 28, towards the grip 16. In squeezing the trigger 18, the leaf spring 20 is compressed. The rotation of the trigger rod 22 about the second pivot 28 brings about movement of the driving rod 24 along the longitudinal axis X of the tube 30. As the trigger rod 22 rotates, the first pivot 26 is able to move freely along the slot 27, such that the driving rod 24 and the first pivot remain positioned along the axis X.

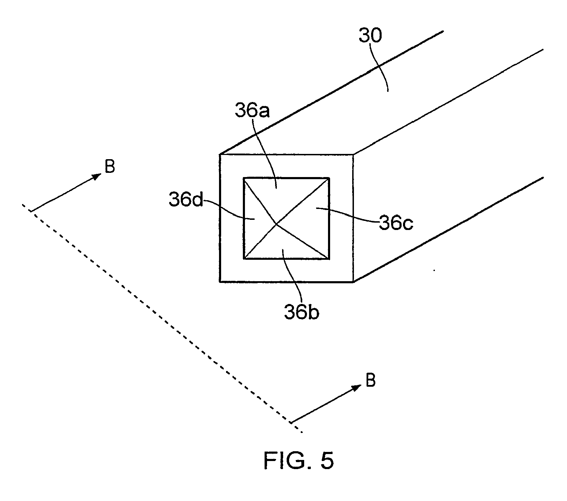

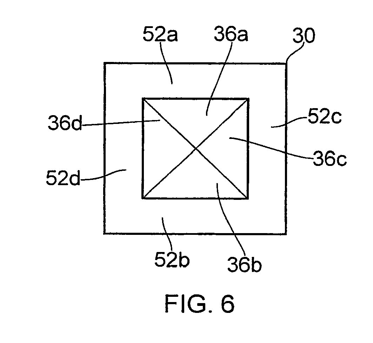

Referring again to Figure 2, as the driving rod 24 moves along the tube 30, so too does the knife member 32. The knife base 34, and each of the blades 36a, 36b, 36c, 36d are advanced towards the opening 48 at the end 50 of the tube 30, which is in contact with the tissue. As the blades 36a, 36b, 36c, 36d advance, the cutting part 40a, 40b, 40c, 40d of each blade engages the corresponding biasing member 52a, 52b, 52c, 52d. Each cutting part 40a, 40b, 40c, 40d is urged inwards towards the axis X of the tube 30. As the cutting parts 40a, 40b, 40c, 40d advance beyond the end 50 of the tube 30, each cutting part pierces the surface of the tissue. Incision into the tissue is achieved with very little resistance, due to the sharpness of the apex of each cutting part 40a, 40b, 40c, 40d. As the blades 36a, 36b, 36c, 36d are further advanced into the tissue, the cutting parts 40a, 40b, 40c, 40d continue to be urged inwards, until, by the time the leading surface 47 of the core member 46 has advanced as far as the opening 48, the apex of each cutting part 40a, 40b, 40c, 40d has converged to a single point. The cutting edges 42a, 44a; 42b, 44b; 42c, 44c; 42d, 44d of adjacent blades engage one another, and the blades 36a, 36b, 36c, 36d form a four-sided pyramid, the base of which is formed by the leading surface 47 of the core member 46. Figures 5 and 6 show the blades 36a, 36b, 36c, 36d forming the walls of a four-sided pyramid extending from the tube 30.

Since, the blades 36a, 36b, 36c, 36d begin to converge before they enter the tissue, they grip the tissue, preventing it from being urged away from the instrument 10 by the advancing blades. Once the blades 36a, 36b, 36c, 36d have converged, and the cutting edges 42a, 44a; 42b, 44b; 42c, 44c; 42d, 44d of adjacent blades have come into contact with one another, the sample is fully cut, and contained within a pyramidal space defined by the blades and the leading surface 47 of the core member 46. The user is then able to withdraw the instrument 10 from the surface of the tissue, with the cleanly-cut sample contained within the pyramidal space. Once clear from the tissue, the user can release his or her grip on the trigger 18. This causes the trigger 18 to be urged away from the grip 16 by the leaf spring 20, thereby causing the driving rod to move along the tube 30 away from the opening 48, and causing the blades 36a, 36b, 36c, 36d to retract back into the tube. In retracting, the blades 36a, 36b, 36c, 36d return to their original straight condition. The sample that was contained within the pyramidal space is then obtainable from the instrument.

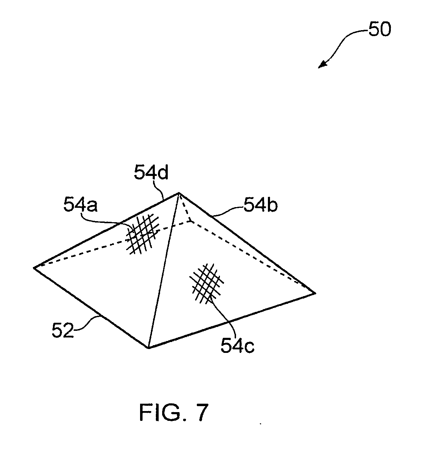

In Figure 7, a four-sided pyramid-shaped sample 50 is shown schematically. The sample, in this case, has a square base 52, formed by the epithelium, and four triangular-shaped sides 54a, 54b, 54c, 54d, which result from the cutting effect of the respective blades 36a, 36b, 36c, 36d.

It is envisaged that, alternatively, an instrument is provided in which a hollow tube has a substantially triangular cross-section. In this embodiment, three blades are provided such that, when they are urged to converge inwards, they form a triangular-based pyramidal space.

Alternatively, the blades 36a, 36b, 36c, 36d may be moved rapidly by a spring from the open configuration to the closed configuration, released by a trigger system as is known to those skilled in the art. Rapid movement of the blades can improve the cut.

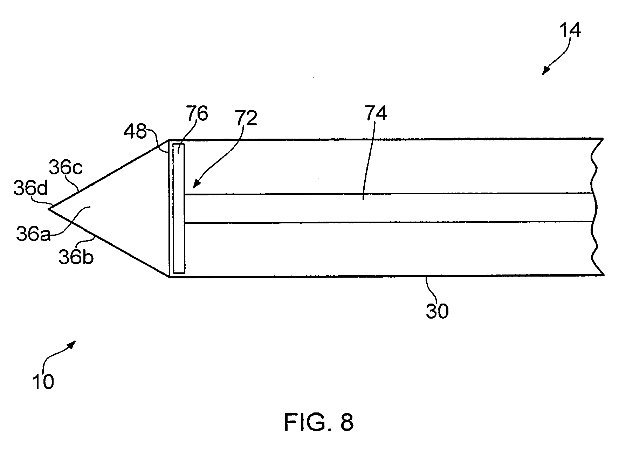

Figure 8 shows a shaft 14 of an instrument 10 constructed in accordance with a second embodiment of the invention. The shaft 14 comprises a hollow tube 30 having a substantially square cross-section. A plunger 72 includes a rod 74 and a substantially square-shaped flange 76 connected to the end of the rod. The plunger 72 is contained within, and moveable along, the tube 30. In its rest configuration, the flange 76 is located at the end 48 of the tube 30. Four triangular blades 36a, 36b, 36c, 36d are connected to the end 48 of the tube 30, and are arranged such that they define a pyramidal space, the base of which is formed by the flange 76. The rod 74 is connected, at its other end, to a driving mechanism, actuatable by a handle (not shown). The handle could be one of the handles described herein with respect to the first embodiment of this invention, or could be any other handle, suitable for actuating such a driving mechanism, known to a person skilled in the art.

Due to the driving mechanism acting on the rod 74, the plunger 72 is moveable from its rest position, towards the blades 36a, 36b, 36c, 36d. As the flange 76 engages with the blades 36a, 36b, 36c, 36d, they are urged outwards, pivoting at their bases, where they attach to the tube 30.

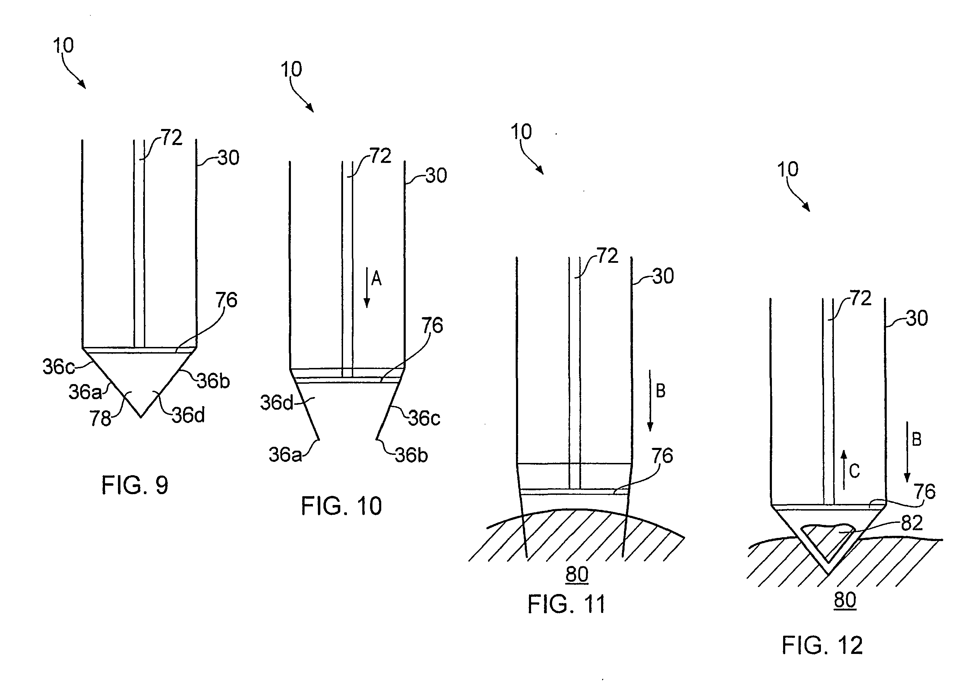

Figures 9 to 12 show, in cross section, how the instrument 10 constructed in accordance with the second embodiment of the invention operates in use. In Figure 9, the shaft end of the instrument 10 is shown. The plunger 72 is in its initial position, with the flange 76 located in line with the bases of the blades 36a, 36b, 36c, 36d. The blades 36a, 36b, 36c, 36d are in a 'closed' configuration, forming with the flange 76, a pyramidal space 78.

As shown in Figure 10, in use, a user causes the plunger 72 to move along the tube 30 in the direction of arrow A, thereby causing the blades 36a, 36b, 36c, 36d to pivot to an 'open' position. With the blades 36a, 36b, 36c, 36d arranged substantially parallel to the walls of the tube 30, as is shown in Figure 11, the instrument 10 is positioned such that the points of the blades are close to the surface of the tissue from which a sample is to be removed. To remove a sample, the user applies force to the instrument 10, in the direction of the tissue 80 (shown by arrow B), causing the blades 36a, 36b, 36c, 36d to pierce the tissue. As the blades 36a, 36b, 36c, 36d are pushed further into the tissue 80, in the direction of the arrow B (shown in Figure 12), the plunger 72 is withdrawn back into the tube 30, in the direction of arrow C. As the plunger 72 is withdrawn, the blades 36a, 36b, 36c, 36d are caused to pivot back to their rest position. By withdrawing the plunger 72 at a rate similar to that at which the blades 36a, 36b, 36c, 36d are pushed into the tissue 80, the user can achieve a smooth, clean cut of the tissue. Once the plunger 72 has been fully withdrawn, and the blades 36a, 36b, 36c, 36d have converged to reform the pyramidal space 78, the instrument 10 is withdrawn from the tissue 80. The plunger 72 is again moved towards the blades 36a, 36b, 36c, 36d, causing them to pivot again to their open position, thereby revealing the sample 82.

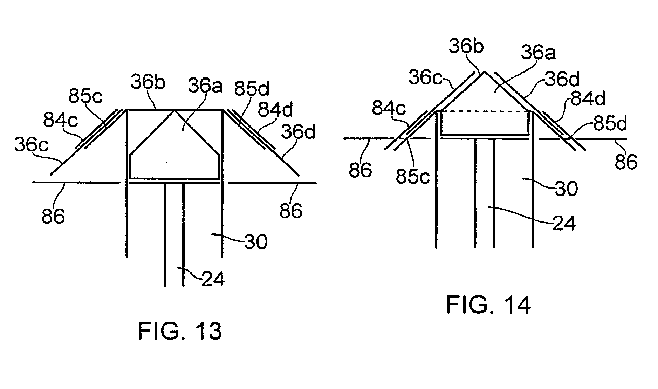

In a third embodiment of the invention, shown in Figures 13 and 14, the blades 36a 36b, 36c, 36d, in a first configuration, are positioned outside the support structure 30. Each blade 36a, 36b, 36c, 36d is positioned between a corresponding pair of guides 84a, 85a; 84b, 85b; 84c, 85c; 84d, 85d, which guide the blades in their transition from the first configuration to the second configuration and vice versa. Guides 84a, 85a, 84b and 85b have been omitted from figures 13 and 14 for clarity. The blades are positioned and angled such that, when the instrument 10 is actuated by a user, each blade moves forward in a straight line, to form a pyramid shape (see Figure 12). The blades are urged forwards by arms 86 extending perpendicularly from the longitudinal axis of the tube 30. When a user actuates the device, the plunger 24 moves along the tube 30, causing the arms 86 to engage the blades, urging them into the second configuration. Of course, other known means for urging the blades into the second configuration will be known to those skilled in the art. In this embodiment, the blades are not required to bend, or to be urged into their second configuration. The blades, therefore, can be made from a stronger, less flexible material.

The instrument may be formed of materials that permit a kink or bend to be generated as needed by the user, by him or her bending the tube 30. Alternatively, the knife 32 may be pivotally connected to the hollow tube 30 to enable the angle of the knife relative to the tube to be varied. The ability of the knife 32 to be pivoted or angled with respect to the tube 30 allows a user to angle the knife in such a way that a piece of tissue can be approached perpendicularly. The pivot means (not shown) may also be provided with a locking means (not shown) for locking the knife 32 in a desired pivoted position.

The support structure 30 may comprise a skeletal frame. Such a frame allows the user to see the site from which a sample is to be taken.

It will be apparent to a person skilled in the art of instrument design that a number of modifications may be made to the instrument described herein without departing from the scope of the appended claims.

The leaf spring 20 may be replaced by any other resilient member capable of urging the trigger 18 to its first position, for example a coil spring.

The means for advancing the blades 36a, 36b, 36c, 36d from within the hollow tube 30 in the first and second embodiments of the invention may be altered. Instead of the gradual, controlled advancement that occurs in the first embodiment, or the rapid advancement caused by a spring-loaded mechanism, the advancement of the blades 36a, 36b, 36c, 36d could be achieved by a graduated mechanism.

## Claims

1. A surgical instrument (10) for removing a sample (50, 82) from a body of tissue (80), the instrument comprising:
a support structure (30); and
a knife (32) arranged to cut into the body of tissue (80), the knife (32) having at least a first blade (36a), a second blade (36b) and a third blade (36c), each blade having two cutting edges (42a, 44a; 42b, 44b; 42c, 44c) defining an apex, and the knife (32) being movable between an open configuration and a closed configuration **characterised in that** the blades (36a, 36b, 36c) define the walls of a pyramidal enclosure for containing the sample (50, 82).

2. An instrument (10) according to claim 1, wherein, in the open configuration, the blades (36a, 36b, 36c) are substantially aligned along a longitudinal axis of at least part of the support structure (30).

3. An instrument (10) according to claim 1 or claim 2, further comprising a biasing member (52a, 52b, 52c) arranged to urge each blade (36a, 36b, 36c) towards the central axis of the support structure.

4. A surgical instrument (10) according to claim 1, wherein each blade (36a, 36b, 36c) remains in the same plane in transition between the open and closed configurations.

5. A surgical instrument (10) according to claim 1 or claim 4, wherein, in the open configuration, the blades (36a, 36b, 36c) are substantially outside the support structure (30).

6. A surgical instrument (10) according to any preceding claim, further comprising actuation means, capable of causing the knife (32) to move between the first configuration and the second configuration.

7. A surgical instrument (10) according to any one of the preceding claims, further comprising locking means for preventing movement of the knife (32) between the open configuration and the closed configuration, and/or between the closed configuration and the open configuration.

8. A surgical instrument (10) according to claim 1, further comprising a plunger (72) disposed in, and being axially movable in a longitudinal direction along, the support structure (30), the plunger (72) being arranged to move the knife (32) between the open configuration and the closed configuration, and/or between the closed configuration and the open configuration.

9. A surgical instrument (10) according to any one of the preceding claims, wherein each blade (36a, 36b, 36c) is diametrically opposite another blade (36a, 36b, 36c).

10. A surgical instrument (10) according to any one of the preceding claims, wherein the knife (32) further comprises a fourth blade, the fourth blade being arranged such that the fourth blade is diametrically opposite to the second blade, and the third blade being diametrically opposite to the first blade.

11. A surgical instrument (10) according to any one of the preceding claims, wherein the support structure (30) is angled close to the knife (32), or is capable of being angled or bent, such that the axis of the knife (32) is, or can be, offset from, or at an angle to, the axis of at least part of the support structure (30).

12. A surgical instrument (10) according to any one of the preceding claims, wherein the knife (32) is pivotable relative to the axis of the support structure (30).

13. A surgical instrument (10) according to claim 11, or claim 12, wherein the knife (32) is pivotable through an angle of between 0 and 45 degrees from the axis of the support structure (30).

14. A surgical instrument (10) according to claim 12 or claim 13, further comprising a securing means for securing the knife (32) in a pivoted position relative to the support structure (30).

## Patentansprüche

1. Chirurgisches Instrument (10) zum Entfernen einer Probe (50, 82) aus einem Gewebekörper (80), wobei das Instrument umfasst:
eine Trägerstruktur (30) und
ein Messer (32), das dazu dient, in den Gewebekörper (80) einzuschneiden, wobei das Messer (32) zumindest eine erste Klinge (36a), eine zweite Klinge (36b) und eine dritte Klinge (36c) umfasst, wobei jede Klinge zwei Schneidkanten (42a, 44a; 42b, 44b; 42c, 44c) aufweist, die eine Spitze bilden, und wobei das Messer (32) zwischen einer geöffneten Konfiguration und einer geschlossenen Konfiguration bewegbar ist, **dadurch gekennzeichnet, dass** die Klingen (36a, 36b, 36c) die Wände einer pyramidenförmigen Einfassung zur Aufnahme der Probe (50, 82) bilden.

2. Instrument (10) nach Anspruch 1, wobei die Klingen (36a, 36b, 36c) in der offenen Konfiguration im Wesentlichen entlang einer Längsachse zumindest eines Teil der Trägerstruktur (30) ausgerichtet sind.

3. Instrument (10) nach Anspruch 1 oder Anspruch 2, wobei dieses ferner ein Vorspannelement (52a, 52b, 52c) umfasst, das dazu dient, die Klingen (36a, 36b, 36c) jeweils in Richtung der Mittelachse der Stützstruktur zu drücken.

4. Chirurgisches Instrument (10) nach Anspruch 1, wobei die Klingen (36a, 36b, 36c) beim Übergang zwischen der offenen und der geschlossenen Konfiguration jeweils in derselben Ebene verbleiben.

5. Chirurgisches Instrument (10) nach Anspruch 1 oder Anspruch 4, wobei sich die Klingen (36a, 36b, 36c) in der offenen Konfiguration im Wesentlichen außerhalb der Trägerstruktur (30) befinden.

6. Chirurgisches Instrument (10) nach einem der vorhergehenden Ansprüche, welches ferner Betätigungsmittel umfasst, mittels derer das Messer (32) zwischen der ersten Konfiguration und der zweiten Konfiguration bewegt werden kann.

7. Chirurgisches Instrument (10) nach einem der vorhergehenden Ansprüche, welches ferner eine Verriegelungseinrichtung umfasst, um eine Bewegung des Messers (32) zwischen der offenen Konfiguration und der geschlossenen Konfiguration und/oder zwischen der geschlossenen Konfiguration und der offenen Konfiguration zu verhindern.

8. Chirurgisches Instrument (10) nach Anspruch 1, welches ferner einen Kolben (72) umfasst, der in der Stützstruktur (30) angeordnet ist und in einer Längsrichtung axial entlang dieser bewegbar ist, wobei der Kolben (72) dazu dient, das Messer (32) zwischen der offenen Konfiguration und der geschlossenen Konfiguration und/oder zwischen der geschlossenen Konfiguration und der offenen Konfiguration zu bewegen.

9. Chirurgisches Instrument (10) nach einem der vorhergehenden Ansprüche, wobei jede Klinge (36a, 36b, 36c) diametral gegenüberliegend einer weiteren Klinge (36a, 36b, 36c) angeordnet ist.

10. Chirurgisches Instrument (10) nach einem der vorhergehenden Ansprüche, wobei das Messer (32) ferner eine vierte Klinge umfasst, wobei die vierte Klinge derart angeordnet ist, dass sich die vierte Klinge diametral gegenüberliegend der zweiten Klinge und die dritte Klinge diametral gegenüberliegend der ersten Klinge befindet.

11. Chirurgisches Instrument (10) nach einem der vorhergehenden Ansprüche, wobei die Trägerstruktur (30) in der Nähe des Messers (32) abgewinkelt ist oder abgewinkelt oder abgebogen werden kann, so dass die Achse des Messers (32) zu der Achse zumindest eines Teils der Trägerstruktur (30) versetzt oder geneigt ist oder versetzt oder geneigt werden kann.

12. Chirurgisches Instrument (10) nach einem der vorhergehenden Ansprüche, wobei das Messer (32) in Bezug auf die Achse der Trägerstruktur (30) schwenkbar ist.

13. Chirurgisches Instrument (10) nach Anspruch 11 oder Anspruch 12, wobei das Messer (32) in einen Winkel zwischen 0 und 45 Grad von der Achse der Stützstruktur (30) weg schwenkbar ist.

14. Chirurgisches Instrument (10) nach Anspruch 12 oder Anspruch 13, welches ferner ein Arretierungsmittel zum Arretieren des Messers (32) in einer in Bezug auf die Trägerstruktur (30) geschwenkten Position umfasst.

## Revendications

1. Instrument chirurgical (10) permettant d'extraire un échantillon (50, 82) d'un corps de tissu (80), cet instrument comprenant :
une structure-support (30), et
un couteau (32) réalisé pour effectuer une découpe dans le corps de tissu (80), ce couteau (32) ayant au moins une première lame (36a), une seconde lame (36b) et une troisième lame (36c), chacune de ces lames ayant deux bords de coupe (42a, 44a, 42b, 44b, 42c, 44c) définissant un sommet en pointe, et le couteau (32) étant mobile entre une configuration ouverte et une configuration fermée, **caractérisé en ce que** les lames (36a, 36b, 36c) définissent les parois d'un réceptacle pyramidal destiné à renfermer l'échantillon (50, 82).

2. Instrument (10) conforme à la revendication 1, dans lequel dans la configuration ouverte, les lames (36a, 36b, 36c) sont essentiellement alignées le long de l'axe longitudinal d'au moins une partie de la structure support (30).

3. Instrument (10) conforme à la revendication 1 ou 2, comprenant en outre un élément de précontrainte (52a, 52b, 52c) réalisé pour rappeler chaque lame (36a, 36b, 36c) vers l'axe central de la structure support.

4. Instrument chirurgical (10) conforme à la revendication 1, dans lequel chaque lame (36a, 36b, 36c) reste dans le même plan lors de la transition entre la configuration ouverte et la configuration fermée.

5. Instrument chirurgical (10) conforme à la revendication 1 ou à la revendication 4, dans lequel, dans la configuration ouverte, les lames (36a, 36b, 36c) sont situées essentiellement à l'extérieur de la structure support (30).

6. Instrument chirurgical (10) conforme à l'une quelconque des revendications précédentes, comprenant en outre des moyens d'activation susceptibles d'entraîner le déplacement du couteau (32) entre la première configuration et la seconde configuration.

7. Instrument chirurgical (10) conforme à l'une quelconque des revendications précédentes, comprenant en outre des moyens de blocage permettant d'empêcher le déplacement du couteau (32) entre la configuration ouverte et la configuration fermée et/ou entre la configuration fermée et la configuration ouverte.

8. Instrument chirurgical (10) conforme à la revendication 1, comprenant en outre un piston (72) monté dans, la structure support (30) et mobile axialement en direction longitudinale le long de celle-ci, ce piston (72) étant réalisé pour déplacer le couteau (32) entre la configuration ouverte et la configuration fermée et/ou entre la configuration fermée et la configuration ouverte.

9. Instrument chirurgical (10) conforme à l'une quelconque des revendications précédentes, dans lequel chaque lame (36a, 36b, 36c) est diamétralement opposée à une autre lame (36a, 36b, 36c).

10. Instrument chirurgical (10) conforme à l'une quelconque des revendications précédentes, dans lequel le couteau (32) comprend en outre une quatrième lame, cette quatrième lame étant réalisée de sorte qu'elle soit diamétralement opposée à la seconde lame, et la troisième lame étant diamétralement opposée à la première lame.

11. Instrument chirurgical (10) conforme à l'une quelconque des revendications précédentes, dans lequel la structure support (30) est pliée à proximité du couteau (32) ou est susceptible d'être pliée ou courbée de sorte que l'axe du couteau (32) soit ou puisse être décalé ou déplacé angulairement par rapport à l'axe d'au moins une partie de la structure support (30).

12. Instrument chirurgical (10) conforme à l'une quelconque des revendications précédentes dans lequel le couteau (32) est susceptible de pivoter par rapport à l'axe de la structure support (30).

13. Instrument chirurgical (10) conforme à la revendication 11 ou 12, dans lequel le couteau (32) est susceptible de pivoter d'un angle compris entre 0 et 45 degrés par rapport à l'axe de la structure support (30).

14. Instrument chirurgical (10) conforme à la revendication 12 ou 13 comprenant en outre des moyens d'arrêt pour permettre de bloquer le couteau (32) dans une position déplacée par pivotement par rapport à la structure support (30).
